# EUROPEAN PATENT APPLICATION

(11) **EP 4 112 060 A1**
(43) Date of publication of application: **04.01.2023**
(21) Application number: 21183145.8
(22) Date of filing: 01.07.2021
(51) Int. Cl.: A61K 31/737, A61P 35/00

(54) **LOW-MOLECULAR-WEIGHT HE800 EXOPOLYSACCHARIDE DERIVATIVES WITH ANTI-CANCER PROPERTIES AND USES THEREOF**

(71) Applicant: Institut Francais de Recherche pour l'Exploitation de la Mer (IFREMER), 92130 Issy-les-Moulineaux (FR); Institut de Cancérologie de l'Ouest (I.C.O), 49100 Angers (FR); Universite de Nantes, 44000 Nantes (FR); Institut National de la Santé et de la Recherche Médicale (INSERM), 75013 Paris (FR)
(72) Inventor: COLLIEC-JOUAULT, Sylvia, 44100 NANTES (FR); SINQUIN, Corinne, 44300 NANTES (FR); MUNOZ GARCIA, Javier, 44850 LIGNE (FR); HEYMANN, Dominique, 44610 INDRE (FR); ZYKWINSKA, Agata, 44800 SAINT HERBLAIN (FR)
(74) Representative: Plasseraud IP

(57) **Abstract**

The invention provides low-molecular-weight non-sulfated or sulfated exopolysaccharide derivatives prepared from a marine native exopolysaccharide excreted by a mesophilic marine bacterium from a deep-sea hydrothermal environment (the HE800 strain, a *Vibrio diabolicus* species of the *Vibrio* genus), and relates to the use of such low-molecular-weight exopolysaccharide derivatives for the treatment of cancer.

## Description

### Background of the Invention

Cancer is among the leading causes of mortality in developed countries. Current major treatments for cancer management include surgery, cytotoxic chemotherapy, targeted therapy, radiation therapy, endocrine therapy, and immunotherapy. Despite the endeavors and achievements made in treating cancers during the past decades, disease recurrence and progression remain a major obstacle to therapy. One of the main clinical issues is the development of drug resistance. Drug resistance exists in two forms: acquired resistance, where the drug is initially efficient but becomes ineffective over time; and intrinsic resistance, which occurs when a drug is ineffective from the beginning of treatment. Many strategies have been designed to combat drug resistance, either by combining the currently available therapies or by developing novel therapies. While the focus is shifting to the development and application of novel therapeutic agents for immunotherapy and targeted therapy, chemotherapy is still standard-of-care in the treatment of most cancers and new and effective chemotherapeutic agents are still needed.

Carbohydrates, and especially heparin or heparan sulfate, are now considered as good candidates to treat cancers. However, their therapeutic use is limited because they both exhibit anticoagulant activity and therefore, they can induce adverse bleeding complications. Another disadvantage of heparin and heparan sulfate is their animal origin, which can result in a high risk of unknown cross-species contamination (Stevenson et al., Research, 2007, 120: S107-S111; Velasco et al., Drug Discov. Today, 2010, 15: 553-560). Consequently, the exploration of the therapeutic potential of heparin mimetics is booming. Sulfated oligosaccharides have been studied, such as a sulfated form of phosphomannopentaose and phosphomannotetraose named PI-88 (Ferro et al., Carbohydr. Res., 2001, 332: 183-189); and a sulfated form of maltohexose and sulfated maltotriose (Vismara et al., Molecules, 2012, 17: 9912-9930). Two polysaccharides extracted from *Prunella vulgaris* L. have also been described for their anti-lung adenocarcinoma activity (Feng et al., Molecules, 2010, 15: 5096-5103).

In recent years, there has been a growing interest in the isolation and identification of new microbial polysaccharides that might have new applications in diverse industries. They compete with polysaccharides from other sources such as seaweeds, crustaceans, animals or plants. Interest in mass culture of microorganisms from the marine environment has increased considerably, representing an innovative approach to the biotechnological use of under-exploited resources. When they are sulfated, polysaccharides from different sources can share some biological properties with glycosaminoglycans (GAGs), and especially heparan sulfate or heparin, without exhibiting the same bleeding risks and with a low risk of contamination by a non-conventional transmissible agent such as prions or emerging viruses due to a large "species-barrier" (DeAngelis, Appl. Microbiol. Biotechnol., 2012, 94: 295-305).

Marine bacteria associated with deep-sea hydrothermal conditions have demonstrated their ability to produce, in an aerobic carbohydrate-based medium, unusual extracellular polymers. They present original structural features that can be modified to design bioactive compounds and improve their specificity (Rehm et al., Rev. Microbiol., 2010, 8: 578-592; Colliec-Jouault et al., Handbook of Exp. Pharmacol., 2012, 423-449). In particular, with the aim of promoting biological activities, chemical modifications (depolymerization and substitution reactions) of the exopolysaccharide HE800 EPS produced by deep-sea a hydrothermal bacterium (*Vibrio diabolicus* of the *Vibrio* genus) have been undertaken. Low-molecular-weight (<25,000 Da) HE800 EPS derivatives thus obtained, which efficiently stimulate fibroblast proliferation, were found to be useful as wound healing agents in the treatment or prevention of diseases of connective tissues, in particular skin and gum tissues (WO 2006/003290).

### Summary of the Invention

The present Inventors have shown that three low-molecular-weight polysaccharides, HE800DR, HE800DRS₂₀ and HE800DRS₃₀, derived from a marine native exopolysaccharide (EPS) excreted by the strain HE800 (a *Vibrio diabolicus* species of the *Vibrio* genus), efficiently reduce cancer cell viability and significantly inhibit cancer cell proliferation in a large variety of human cancer cell lines including osteosarcoma, lung cancer, breast cancer, melanoma and colon cancer cell lines. The three low-molecular-weight HE800 EPS derivatives have the same molecular weight (20,000 Da) but different degrees of sulfate-group substitution (0% for HE800DR, 20% for HE800DRS₂₀ and 30% for HE800DRS₃₀, by weight relative to the total weight of the polysaccharide).

Accordingly, in a first aspect, the present invention relates to a low-molecular-weight exopolysaccharide derivative for use in the treatment of cancer in a subject, wherein said low-molecular-weight exopolysaccharide derivative is obtained by a method comprising:
(a) a step consisting of free-radical depolymerization of a marine native exopolysaccharide (EPS) from the strain HE800 of the *Vibrio diabolicus* genus so as to obtain a depolymerized EPS having a molecular weight of 5,000 to 100,000 g/mol; and
(b) a subsequent step consisting of isolating a low-molecular-weight exopolysaccharide derivative from the depolymerized EPS, wherein the low-molecular-weight exopolysaccharide derivative has a molecular weight comprised between 5,000 and 30,000 g/mol, preferably between 10,000 and 25,000 g/mol, more preferably between 15,000 and 22,000 g/mol, and even more preferably a molecular weight of about 20,000 g/mol.
or wherein said low-molecular-weight exopolysaccharide derivative is a low-molecular-weight sulfated exopolysaccharide derivative and said low-molecular-weight sulfated exopolysaccharide derivative is obtained by a method comprising:
(a') a step consisting of free-radical depolymerization of a marine native exopolysaccharide (EPS) from the strain HE800 of the *Vibrio diabolicus* genus so as to obtain a depolymerized EPS having a molecular weight of 5,000 to 100,000 g/mol;
(b') a subsequent step consisting of sulfation of the depolymerized EPS to obtain a sulfated depolymerized EPS, comprising adding to the depolymerized EPS at least one sulfation agent in an amount sufficient to obtain a sulfated polysaccharide having a degree of sulfate-group substitution of between 5% and 40% by weight relative to the total weight of the sulfated depolymerized EPS; and
(c') a subsequent step consisting of isolating the low-molecular-weight sulfated exopolysaccharide derivative from the sulfated depolymerized EPS, wherein the low-molecular-weight sulfated exopolysaccharide derivative has a molecular weight comprised between 5,000 and 30,000 g/mol, preferably between 10,000 and 25,000 g/mol, more preferably between 15,000 and 22,000 g/mol, and even more preferably a molecular weight of about 20,000 g/mol.

In certain embodiments, the method used for obtaining the low-molecular-weight exopolysaccharide derivative is such that step (b) is carried out by fractionation, in particular fractionation performed by size exclusion chromatography.

In other embodiments, the method used for obtaining the low-molecular-weight sulfated exopolysaccharide derivative is such that step (c') is carried out by fractionation, in particular fractionation performed by size exclusion chromatography.

In certain particular embodiments, the low-molecular-weight exopolysaccharide derivative is HE800DR, which has a molecular weight of 20,000 g/mol and a degree of sulfate-group substitution of 0% by weight relative to the total weight of the depolymerized EPS.

In other particular embodiments, the low-molecular-weight exopolysaccharide derivative is the low-molecular-weight sulfated exopolysaccharide derivative, HE800DRS₂₀, which has a molecular weight of 20,000 g/mol and a degree of sulfate-group substitution of 20% by weight relative to the total weight of the sulfated depolymerized EPS.

In yet other particular embodiments, the low-molecular-weight exopolysaccharide derivative is the low-molecular-weight sulfated exopolysaccharide derivative, HE800DRS₃₀, which has a molecular weight of 20,000 g/mol and a degree of sulfate-group substitution of 30% by weight relative to the total weight of the sulfated depolymerized EPS.

In certain embodiments, a low-molecular-weight exopolysaccharide derivative, as described herein, used in the treatment of cancer, wherein the cancer is selected from the group consisting of carcinoma, lymphoma, blastoma, sarcoma, and leukemia.

The cancer may be selected from the group consisting of bone cancer, lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, and pituitary adenoma.

In certain embodiments, the cancer is a solid malignant tumor. In particular, the solid cancer may be selected from the group consisting of osteosarcoma, lung cancer, breast cancer, melanoma and colon cancer.

In certain embodiments, the cancer is metastatic cancer.

In certain particular embodiments, the low-molecular-weight exopolysaccharide derivative is HE800DR, HE800DRS₂₀ or HE800DRS₃₀ and the cancer is selected from the group consisting of osteosarcoma, lung cancer, breast cancer, melanoma and colon cancer. In certain particular embodiments, the low-molecular-weight exopolysaccharide derivative is HE800DR and the cancer is selected from lung cancer and melanoma. In other particular embodiments, the low-molecular-weight exopolysaccharide derivative is HE800DRS₂₀ and the cancer is osteosarcoma. In yet other particular embodiments, the low-molecular-weight exopolysaccharide derivative is HE800DRS₃₀ and the cancer is osteosarcoma, breast cancer or colon cancer.

In certain preferred embodiments, the subject is a cancer patient. A cancer patient may be suffering from a cancer, or may have previously undergone therapy for cancer. When the cancer patient is suffering from a cancer, the cancer patient may be undergoing therapy for cancer.

In the same first aspect, the present invention provides a method for treating cancer in a subject, the method comprising a step of administering to said subject in need thereof a therapeutically effective amount of a low-molecular-weight exopolysaccharide derivative as defined herein. In particular, the low-molecular-weight exopolysaccharide derivative may be HE800DR, HE800DRS₂₀ or HE800DRS₃₀. In certain preferred embodiments of the method of treatment of the invention, the subject is a cancer patient, as described above. In certain preferred embodiments, the cancer to be treated is as described above.

In another aspect, the present invention provides a pharmaceutical composition comprising an effective amount of a low-molecular-weight exopolysaccharide derivative, as defined herein, and at least one pharmaceutically acceptable carrier or excipient for use in the treatment of cancer in a subject. For example, the low-molecular-weight exopolysaccharide derivative that is present in a pharmaceutical composition may be HE800DR, HE800DRS₂₀ or HE800DRS₃₀. In certain preferred embodiments, the subject to be treated with a pharmaceutical composition described herein is a cancer patient, as described above. In certain preferred embodiments, the cancer to be treated is as described above.

These and other objects, advantages and features of the present invention will become apparent to those of ordinary skill in the art having read the following detailed description of the preferred embodiments.

### Brief Description of the Figures

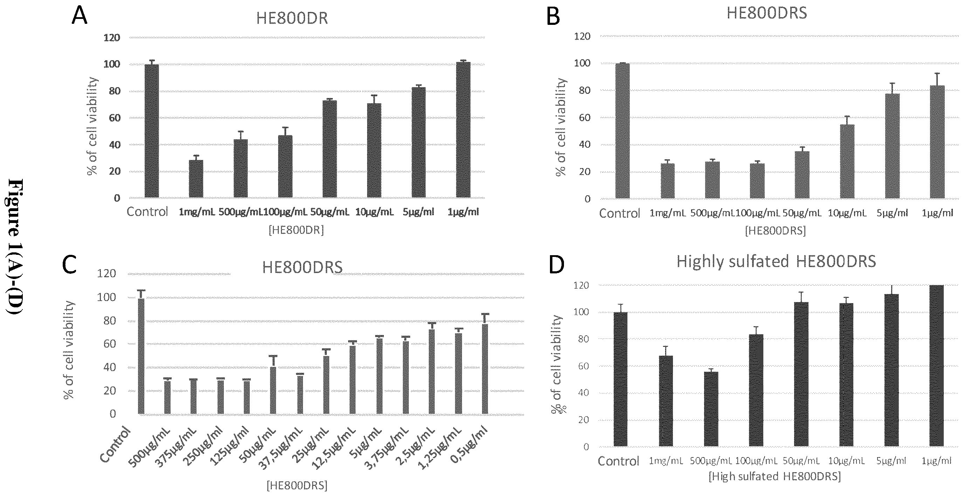

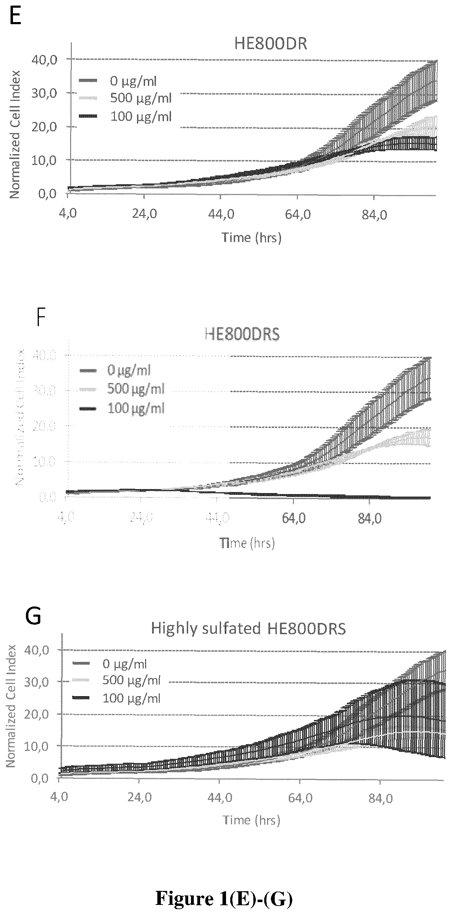

**Figure 1****:** Effect of low-molecular-weight HE800 exopolysaccharides in human MNNG/HOS osteosarcoma cell line. Cell viability in the presence of (A) HE800DR, (**B**) and (**C**) HE800DRS (*i.e.,* HE800DRS₂₀) and (**D**) highly sulfated HE800DRS (*i.e.,* HE800DRS₃₀) at different concentrations, and normalized cell index in the presence of **(E)** HE800DR, **(F)** HE800DRS and **(G)** highly sulfated HE800DRS as a function of time.

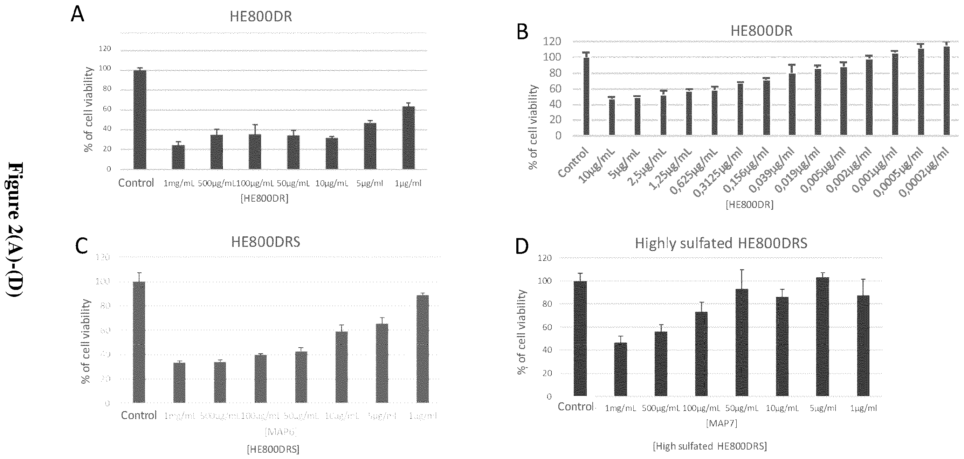

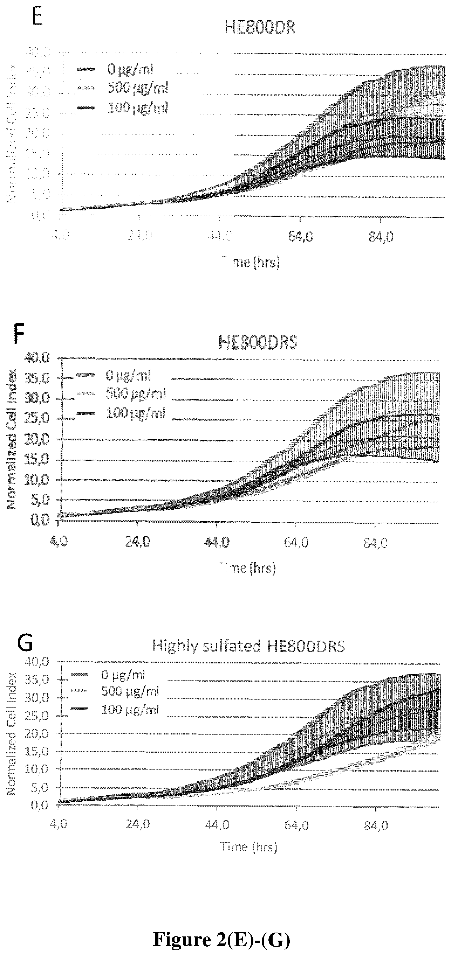

**Figure 2****.** Effect of low-molecular-weight HE800 exopolysaccharides in human A549 lung cancer cell line. Cell viability in the presence of **(A)** and **(B)** HE800DR, **(C)** HE800DRS (*i.e.,* HE800DRS₂₀) and **(D)** highly sulfated HE800DRS (*i.e.,* HE800DRS₃₀) at different concentrations, and normalized cell index in the presence of **(E)** HE800DR, **(F)** HE800DRS and **(G)** highly sulfated HE800DRS as a function of time.

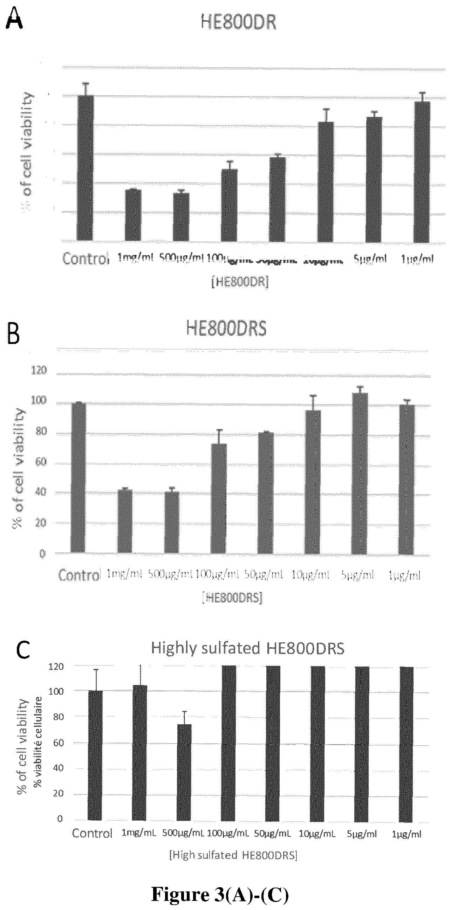

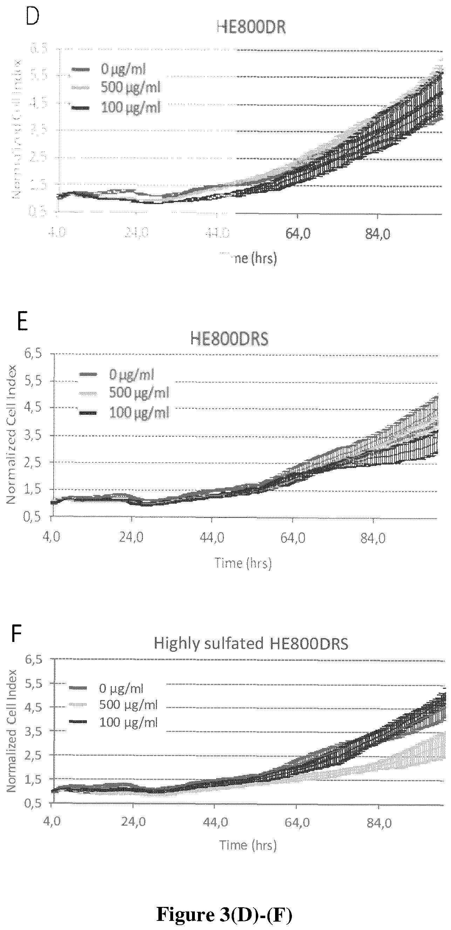

**Figure 3****.** Effect of low-molecular-weight HE800 exopolysaccharides in human MDA-MB-231 breast cancer cell line. Cell viability in the presence of **(A)** HE800DR, **(B)** HE800DRS (*i.e.,* HE800DRS₂₀) and **(C)** highly sulfated HE800DRS (*i.e.,* HE800DRS₃₀) at different concentrations, and normalized cell index in the presence of **(D)** HE800DR, **(E)** HE800DRS and **(F)** highly sulfated HE800DRS as a function of time.

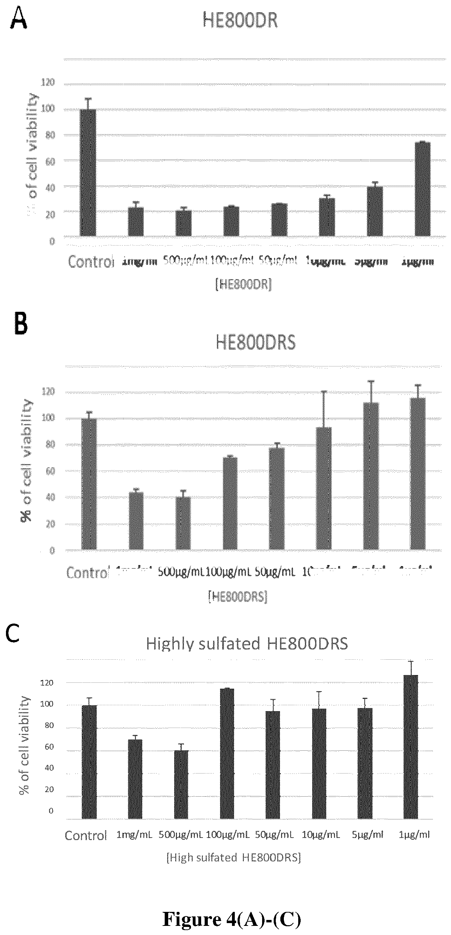

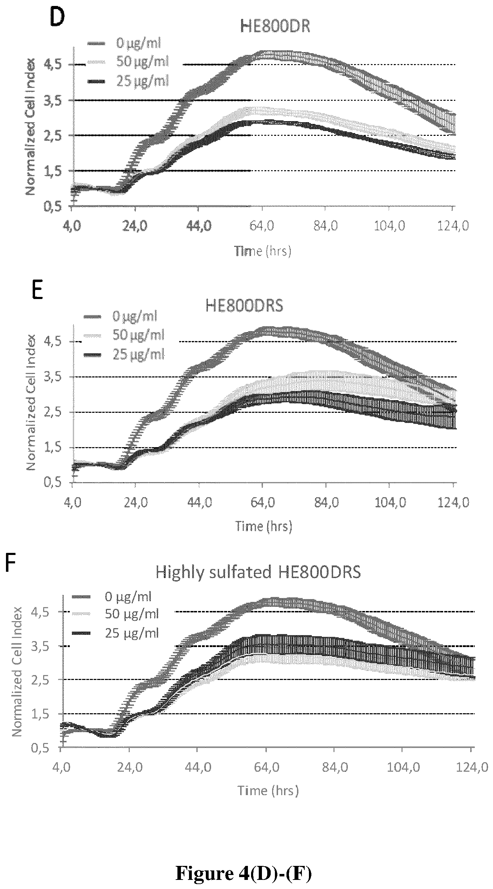

**Figure 4****.** Effect of low-molecular-weight HE800 exopolysaccharides in human A375 melanoma cell line. Cell viability in the presence of **(A)** HE800DR, **(B)** HE800DRS (*i.e.,* HE800DRS₂₀) and **(C)** highly sulfated HE800DRS (*i.e.,* HE800DRS₃₀) at different concentrations, and normalized cell index in the presence of **(D)** HE800DR, **(E)** HE800DRS and **(F)** highly sulfated HE800DRS as a function of time.

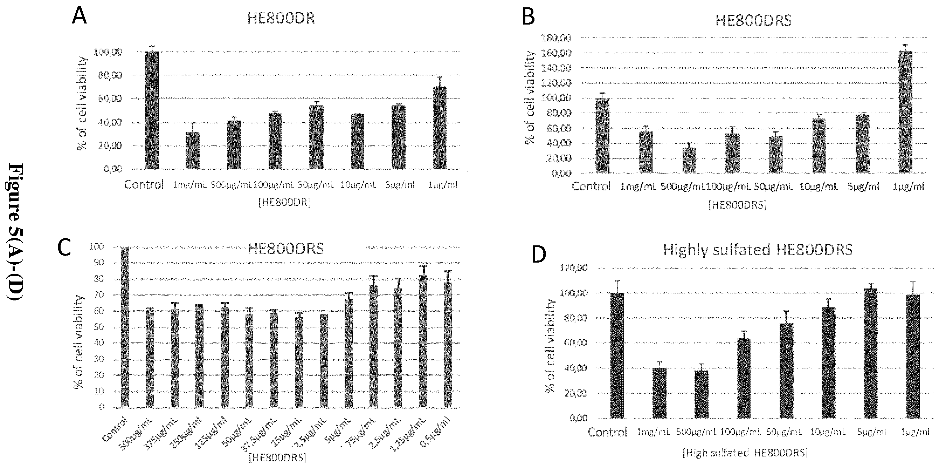

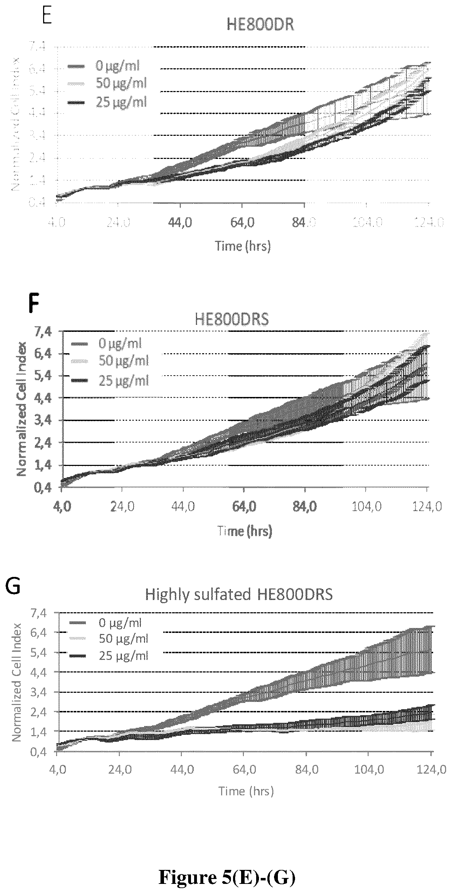

**Figure 5****.** Effect of low-molecular-weight HE800 exopolysaccharides in human Caco2 colon cancer cell line. Cell viability in the presence of **(A)** HE800DR, **(B)** and **(C)** HE800DRS (*i.e.,* HE800DRS₂₀) and **(D)** highly sulfated HE800DRS (*i.e.,* HE800DRS₃₀) at different concentrations, and normalized cell index in the presence of **(E)** HE800DR, **(F)** HE800DRS and **(G)** highly sulfated HE800DRS as a function of time.

### Definitions

As used herein, the term ***"subject"*** refers to a human or another mammal (e.g., primate, dog, cat, goat, horse, pig, mouse, rat, rabbit, and the like), that can develop a cancer, but may or may not be suffering from the disease. Non-human subjects may be transgenic or otherwise modified animals. In many embodiments of the present invention, the subject is a human being. In such embodiments, the subject is often referred to as an *"individual"* or a ***"patient".*** These terms do not denote a particular age, and thus encompass newborns, children, teenagers, and adults. The term *"patient"* more specifically refers to an individual suffering from a disease. Thus, the term *"cancer patient"* refers to an individual suffering from a cancer. A cancer patient may or may not have been diagnosed with cancer. The term also includes individuals that have previously undergone therapy for cancer.

As used herein, the term ***"cancer"*** refers to or describes the physiological condition in mammals that is typically characterized by unregulated cell growth, lack of differentiation and ability to invade local tissues and metastasize. Cancer can develop in any tissue of any organ. Examples of cancers include, but are not limited to carcinoma, lymphoma, blastoma, sarcoma, and leukemia. More particularly, examples of such cancers include bone cancer, lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the pelvis, neoplasms of the central nervous system (CNS), neuroectodermal cancer, spinal axis tumors, glioma, meningioma, and pituitary adenoma.

The terms ***"aggressive"*** and ***"invasive"*** are used herein interchangeably. When used herein to characterize a cancer, they refer to the proclivity of a tumor for expanding beyond its boundaries into adjacent tissue. Invasive cancer can be contrasted with organ-confined cancer wherein the tumor is confined to a particular organ. The invasive property of a tumor is often accompanied by the elaboration of proteolytic enzymes, such as collagenases, that degrade matrix material and basement membrane material to enable the tumor to expand beyond the confines of the capsule, and beyond confines of the particular tissue in which that tumor is located.

The term ***"metastasis",*** as used herein, refers to the spread of tumor cells from one organ or tissue to another location. The term also refers to tumor tissue that forms in a new location as a result of metastasis. A "metastatic cancer" is a cancer that spreads from its original, or primary, location, and may also be referred to as a "secondary cancer" or "secondary tumor". Generally, metastatic tumors are named for the tissue of the primary tumor from which they originate. The process of tumor metastasis is a multistage event involving local invasion and destruction of intercellular matrix, intravasation into blood vessels, lymphatics or other channels of transport, survival in the circulation, extravasation out of the vessels in the secondary site and growth in the new location.

The term ***"treatment"*** is used herein to characterize a method or process that is aimed at (1) delaying or preventing the onset of a disease or condition (here a cancer); (2) slowing down or stopping the progression, aggravation, or deterioration of the symptoms of the disease or condition; (3) bringing about amelioration of the symptoms of the disease or condition; or (4) curing the disease or condition. A treatment may be administered after initiation of the disease or condition, for a therapeutic action. Alternatively, a treatment may be administered prior to the onset of the disease or condition, for a prophylactic or preventive action. In this case, the term ***"prevention"*** is used.

A ***"pharmaceutical composition"*** is defined herein as comprising an effective amount of the low-molecular-weight exopolysaccharide described herein, and at least one pharmaceutically acceptable carrier or excipient.

As used herein, the term ***"effective amount"*** refers to any amount of a compound, agent, or composition that is sufficient to fulfil its intended purpose(s), *e.g.,* a desired biological or medicinal response in a cell, tissue, system or subject.

The term ***"pharmaceutically acceptable carrier or excipient"*** refers to a carrier medium which does not interfere with the effectiveness of the biological activity of the active ingredient(s) and which is not excessively toxic to the host at the concentration at which it is administered. The term includes solvents, dispersion media, coatings, antibacterial and antifungal agents, isotonic agents, and adsorption delaying agents, and the like. The use of such media and agents for pharmaceutically active substances is well known in the art (see for example *"*Remington's Pharmaceutical Sciences", E.W. Martin, 18th Ed., 1990, Mack Publishing Co.: Easton, PA, which is incorporated herein by reference in its entirety).

The terms ***"approximately"*** and ***"about"**,* as used herein in reference to a number, generally include numbers that fall within a range of 10% in either direction of the number (greater than or less than the number) unless otherwise stated or otherwise evident from the context (except where such number would exceed 100% of a possible value).

### Detailed Description of Certain Preferred Embodiments

As mentioned above, the present invention provides low-molecular-weight derivatives of the native HE800 exopolysaccharide that exhibit anti-cancer properties, and the use of the low-molecular-weight HE800 exopolysaccharide derivatives in the treatment of cancer.

### I - Low-Molecular-Weight HE800 Exopolysaccharide Derivatives

The low-molecular-weight (LMW) exopolysaccharide derivatives used in the present invention are derived from a native exopolysaccharide (EPS), HE800 EPS, that is excreted by a mesophilic marine bacterium from a deep-sea hydrothermal environment. In recent years, there has been a growing interest in the isolation and identification of new polysaccharides of marine origin. Marine bacterial EPSs and derivatives thereof have some great advantages as therapeutic compounds because they can be produced at viable economic cost, in controlled conditions in agreement with Good Manufacturing Practices and exhibit a very low risk for patients to be infected by a non-conventional transmissible agent, such as prions or emerging viruses, due to a large "species-barrier".

Marine bacteria from deep-sea hydrothermal vent environments, belonging to three main genera (*Vibrio, Alteromonas* and *Pseudoalteromonas*)*,* have demonstrated their ability to produce unusual extracellular polymers in an aerobic carbohydrate-supplemented medium. The excreted exopolysaccharides present original structural features that can be modified to design bioactive compounds and improve their specificity (Rehm et al., Rev. Microbiol., 2010, 8: 578-592; Colliec-Jouault et al., Handbook of Exp. Pharmacol., 2012, 423-449; Delbarre-Ladrat et al., Microorganisms, 2017, 5(3): 53). In particular, the first EPS-producing species of *Vibrio* to be isolated from an active deep-sea hydrothermal vent sample was named *Vibrio diabolicus* (Raguenes et al., Int. J. Syst. Bacteriol., 1997, 47: 989-995). It produces a high molecular weight (> 10⁶ g/mol - Rougeaux et al., J. Carbohydr. Res., 1999, 322: 40-45) exopolysaccharide, called HE800 EPS, which consists of a linear tretrasaccharide repeating unit: two glucuronic acid residues, one *N*-acetylated glucosamine residue and one *N*-acetylated galactosamine residue.

LMW EPS derivatives and LMW sulfated EPS derivatives from the marine native exopolysaccharide, HE800 EPS, have previously been prepared by the present Inventors (WO 2006/003290; WO 2007/066009; WO 02/002051 ; Senni et al., Mar. Drugs, 2011, 9: 1664-1681; Senni et al., Mar. Drugs, 2013, 11: 1351-1369; Heymann et al., Molecules, 2016, 21: 309). The LMW EPS derivatives and LMW sulfated EPS derivatives are prepared using a step of radical depolymerization and a first step of radical depolymerization followed by a sulfation reaction, respectively, thereby generating bioactive molecules having a molecular weight < 30 kg/mol (30,000 Da).

In the practice of the present invention, a LMW EPS derivative is prepared using a method comprising:
(a) a step consisting of free-radical depolymerization of a marine native EPS from the strain HE800 of the *Vibrio diabolicus* genus so as to obtain a depolymerized EPS having a molecular weight of 5,000 to 100,000 g/mol; and
(b) a subsequent step consisting of isolating a LMW EPS derivative from the depolymerized EPS, wherein the LMW EPS derivative has a molecular weight comprised between 5,000 and 30,000 g/mol, preferably between 10,000 and 25,000 g/mol, more preferably between 15,000 and 22,000 g/mol, and even more preferably a molecular weight of about 20,000 g/mol.

In the practice of the present invention, a LMW sulfated EPS derivative is prepared using a method comprising:
(a') a step consisting of free-radical depolymerization of a marine native EPS from the strain HE800 of the *Vibrio diabolicus* genus so as to obtain a depolymerized EPS having a molecular weight of 5,000 to 100,000 g/mol;
(b') a subsequent step consisting of sulfation of the depolymerized EPS to obtain a sulfated depolymerized EPS, comprising adding to the depolymerized EPS at least one sulfation agent in an amount sufficient to obtain a sulfated polysaccharide having a degree of sulfate-group substitution of between 5% and 40% by weight relative to the total weight of the sulfated depolymerized EPS; and
(c') a subsequent step consisting of isolating a LMW sulfated EPS derivative from the sulfated depolymerized EPS, wherein the LMW sulfated EPS derivative has a molecular weight comprised between 5,000 and 30,000 g/mol, preferably between 10,000 and 25,000 g/mol, more preferably between 15,000 and 22,000 g/mol, and even more preferably a molecular weight of about 20,000 g/mol.

In certain embodiments, the depolymerized derivatives obtained at the end of step (a) or the end of step (a') are lyophilized.

In other embodiments, step (b') of the process is followed by a dialysis step.

In the free-radical depolymerization step (step (a) or step (a')), the native HE800 EPS can be used in a liquid form, *i.e.* as it is excreted by the bacteria into the culture medium. Preferably, the culture medium is centrifuged and only the supernatant containing the native HE800 EPS and that is free of bacterial debris is collected. The native HE800 EPS can be collected by any suitable technique known to those skilled in the art, such as for example membrane ultrafiltration, and can then optionally be lyophilized as is or in the form of an addition salt.

The step consisting of free-radical depolymerization (step (a) or step (a')) of the native HE800 EPS is preferably carried out by adding a solution of an oxidizing agent to a reaction mixture comprising the native HE800 EPS, preferably in the presence of a metal catalyst. The oxidizing agent is preferably chosen from peroxides, in particular hydrogen peroxide, and peracids, especially peracetic acid and 3-chloroperbenzoic acid. The addition is preferably carried out continuously and with stirring for a period of between 30 minutes and 10 hours. The reaction mixture is preferably maintained at a pH of between 6 and 8, for example by addition of a basifying agent such as sodium hydroxide, and at a temperature of between approximately 30°C and 70°C throughout the duration of the free-radical depolymerization reaction.

According to a specific embodiment of the present invention, in this step, the native HE800 EPS is present in the reaction mixture at a concentration of between about 2 mg/ml and about 10 mg/ml of reaction mixture.

In preferred embodiments, the oxidizing agent is a solution of hydrogen peroxide (H₂O₂) preferably having a concentration of between about 0.1% and about 0.5% by weight, preferably of the order of 0.1% to 0.2% by weight, and is added at a flow rate of V1/1000 to V1/10 ml/minute, preferably V1/50 and V1/500 ml/minute, and more preferably of the order of V1/100 ml/minute, wherein V1 is the volume of the reaction medium containing the marine exopolysaccharide to which a solution of hydrogen peroxide is added.

The metal catalysts that can be used during the free-radical depolymerization step are preferably chosen from Cu²⁺, Fe²⁺ and Cr³⁺ ions and the Cr₂O₇²⁻ anion, as described in particular in patent application EP 0 221 977. According to a specific embodiment, the metal catalyst is present in the reaction mixture at a concentration of between about 10⁻³ M and about 10⁻¹ M, and preferably at a concentration of between about 0.001 M and about 0.05 M.

The free-radical depolymerization process, as described above, makes it possible to obtain, in a single step and with a good yield, homogeneous LMW EPS derivatives. In the context of the present invention, the term "homogeneous derivatives" is intended to mean derivatives which, when assessed using high performance size exclusion chromatography, exhibit a single main peak representing a predominant population of polysaccharide chains that are homogeneous with respect to size, characterized by a polydispersity index I (Mw/Mn) < 5, where Mw is the weight-average molecular weight and Mn is the number-average molecular weight.

In certain embodiments, when the depolymerization reaction is over, the depolymerized EPSs obtained are reduced using a reducing agent, so as to stabilize the chains, the reducing ends of which are very reactive, and in particular to avoid chain hydrolysis by the "peeling" reaction. The nature of the reducing agents that can be used to this effect is not essential. In particular, the reducing agent may be sodium borohydride.

The metal catalyst used in the free-radical depolymerization step can be eliminated at the end of the depolymerization reaction, (or at the end of the reduction reaction if a reduction step is carried out) using any suitable method, for example by ion exchange chromatography, preferably a weak cation exchange resin passivated beforehand, or by treatment with EDTA (ethylenediaminetetraacetic acid).

The depolymerized EPSs resulting from the free-radical depolymerization (optionally followed by a reduction step) can, if necessary, be recovered using any suitable technique well known to those skilled in the art, such as, for example, by membrane ultrafiltration or dialysis. Then, they are lyophilized and fractionated by size exclusion chromatography to increase their purity required to improve the subsequent sulfation step (if any). Finally, the purified depolymerized EPSs are conditioned in salt form by addition of a weak or strong base that may be chosen, for example, from pyridine, triethylamine, tributylamine, tetrabutylammonium hydroxide and sodium hydroxide. This lyophilized salt may be prepared, for example, by elution of an aqueous solution of the polysaccharide derivatives at a concentration of between 1 and 8 mg/ml on an ion exchange resin column such as, for example, those sold under the name DOWEX^{®} by the company Dow Chemical. The eluate is collected as long as the pH remains acid, for example less than 5, then the pH is subsequently adjusted to approximately 6.5 with the desired base as defined above. The EPS derivatives in the form of a salt are then ultrafiltered and lyophilized.

When LMW sulfated EPS derivatives are prepared, the lyophilized EPS derivatives, possibly in the form of an addition salt, are preferably dissolved in an anhydrous solvent at the beginning of the sulfation step (step (b')). The solvent is preferably chosen from dimethylformamide (DMF), dimethyl sulfoxide (DMSO) formamide, and mixtures thereof. The amount of EPS derivatives present in the anhydrous solvent may be between approximately 1 and 10 mg/ml, preferably between about 1 mg/ml and about 5 mg/ml, and even more preferably this amount is about 2.5 mg/ml. The dissolution of the EPS in the anhydrous solvent is preferably carried out, with stirring, at ambient temperature for about 1 hour to about 2 hours and then at a temperature of between 40°C and 50°C, preferably at a temperature of about 45°C for about 2 hours under argon or azote with molecular sieve.

The one or more chemical sulfation agents used during the sulfation step can be added to the depolymerized and/or reduced EPSs that are in lyophilized form or in the form of a solution.

The sulfation agents are preferably chosen from complexes of pyridine sulfate (free or coupled to a polymer), of dimethylformamide sulfate, triethylamine sulfate and of trimethylamine sulfate. The one or more chemical sulfation agents are added to the solution of EPS derivatives in a weight amount preferably representing from about 4 to about 6 times, and even more preferably about 5 times, the mass of EPS derivatives in solution. The chemical sulfation reaction is then preferably carried out with stirring for a period of between 2 and 24 hours depending on the desired degree of sulfation. When the desired degree of sulfation is reached, the sulfation reaction is stopped after cooling of the reaction medium:
- either by precipitation in the presence of sodium-chloride-saturated acetone or of methanol, and then dissolution of the precipitate in water;
- or, preferably, by addition of water in a proportion preferably equal to 1/10 of the reaction volume and adjustment of the pH of the reaction medium to 9 with a basifying agent such as, for example, sodium hydroxide (3 M).

The chemical sulfation reaction is continued until the degree of sulfation (or degree of sulfate-group substitution) reaches a value comprised between 5% and 40% by weight relative to the total weight of the sulfated depolymerized EPS, for example about 5%, or about 10%, or about 15%, or about 20%, or about 25% or about 30% or about 35%, or about 40% by weight relative to the total weight of the sulfated depolymerized EPS. In certain embodiments, the chemical sulfation reaction is continued until the degree of sulfation reaches a value of 20% by weight relative to the total weight of the sulfated depolymerized EPS. In other embodiments, the chemical sulfation reaction is continued until the degree of sulfation reaches a value of 30% by weight relative to the total weight of the sulfated depolymerized EPS.

According to certain embodiments, the solution of sulfated EPS derivatives is preferably dialyzed in order to remove the various salts, and then lyophilized. The final product, typically with an accurate molecular weight and a low polydispersity index, is obtained by isolation from the LMW depolymerized EPS obtained in step (a) or from the LMW sulfated depolymerized EPS obtained in step (b'). Isolation (step (b) or step (c')) may be performed by any suitable method known in the art. Preferably, isolation is carried out by fractionation performed by size exclusion chromatography.

The LMW EPS derivative obtained after isolation (step (b)), or the LMW sulfated EPS derivative obtained after isolation (step (c')), has a molecular weight that is comprised between 5,000 and 30,000 g/mol, preferably between 10,000 and 25,000 g/mol, more preferably between 15,000 and 22,000 g/mol, and even more preferably a molecular weight of about 20,000 g/mol. In certain preferred embodiments, the LMW EPS derivative obtained after isolation (step (b)), or the LMW sulfated EPS derivative obtained after isolation (step (c')), has a molecular weight of 20,000 g/ml.

The LMW EPS derivative obtained after isolation (step (b)), or the LMW sulfated EPS derivative obtained after isolation (step (c')), has a low polydispersity index of less than 5, preferably of 1.5 to 4, more preferably of less than 2. The polydispersity index (PDI) is a measure of the distribution of molecular mass of the derivatives. The PDI calculated is the weight average molecular weight divided by the number average molecular weight. PDI is typically measured by size-exclusion chromatography.

In certain particular embodiments, the LMW EPS derivative is HE800DR, which has a molecular weight of 20,000 g/mol and degree of sulfate-group substitution of 0% by weight relative to the total weight of the sulfated depolymerized EPS.

In other particular embodiments, the LMW EPS derivative is the LMW sulfated EPS derivative, HE800DRS₂₀, which has a molecular weight of 20,000 g/mol and a degree of sulfate-group substitution of 20% by weight relative to the total weight of the sulfated depolymerized EPS. HE800DRS₂₀ is also designed herein by the term "HE800DRS" (in particular in the Experimental section and in the Figures).

In yet other particular embodiments, the LMW EPS derivative is the LMW sulfated EPS derivative, HE800DRS₃₀, which has a molecular weight of 20,000 g/mol and a degree of sulfate-group substitution of 30% by weight relative to the total weight of the sulfated depolymerized EPS. HE800DRS₃₀ is also designed herein by the term highly sulfated HE800DRS (in particular in the Experimental section and in the Figures).

### II - Uses of the Low-Molecular-Weight HE800 Exopolysaccharide Derivatives

### Therapeutic Applications

### A. Indications

Due to their ability to efficiently reduce cancer cell viability and to significantly inhibit cancer cell proliferation in a large variety of human cancer cell lines, a LMW EPS derivative (for example HE800DR) or a LMW sulfated EPS derivative (for example HE800DRS₂₀ or HE800DRS₃₀), as described herein, may be used in the treatment of cancer in a subject.

A treatment of cancer in a subject according to the present invention may be accomplished using a LMW EPS derivative or a LMW sulfated EPS derivative, as described herein, or a pharmaceutical composition thereof. These methods generally comprise administration of an effective amount of a LMW EPS derivative or a LMW sulfated EPS derivative, as described herein, or a pharmaceutical composition thereof, to a subject in need thereof. Administration may be performed using any of the methods known to one skilled in the art. In particular, a LMW EPS derivative or a LMW sulfated EPS derivative, as described herein, or a pharmaceutical composition thereof, may be administered by any of various routes including, but not limited to, aerosol, parenteral, oral or topical route.

Generally, the subject is a human cancer patient. The cancer patient may be suffering from a cancer or having previously undergone therapy of cancer.

In the practice of the present invention, the cancer may be any cancer developed in any tissue of any organ. Thus, the cancer may be a carcinoma, lymphoma, blastoma, sarcoma, and leukemia. Examples of cancers include, but are not limited to, bone cancer, lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the pelvis, neoplasms of the central nervous system (CNS), neuroectodermal cancer, spinal axis tumors, glioma, meningioma, and pituitary adenoma.

In certain embodiments, the cancer is a solid malignant tumor. In particular, the solid cancer may be selected from the group consisting of osteosarcoma, lung cancer, breast cancer, melanoma and colon cancer.

In certain embodiments, the cancer is metastatic cancer.

In certain particular embodiments, the LMW EPS derivative is HE800DR, HE800DRS₂₀ or HE800DRS₃₀ and the cancer is selected from the group consisting of osteosarcoma, lung cancer, breast cancer, melanoma and colon cancer. In certain particular embodiments, the LMW EPS derivative is HE800DR and the cancer is selected from lung cancer and melanoma. In other particular embodiments, the LMW EPS derivative is the LMW sulfated EPS derivative HE800DRS₂₀ and the cancer is osteosarcoma. In yet other particular embodiments, the LMW EPS derivative is the LMW sulfated EPS derivative HE800DRS₃₀ and the cancer is osteosarcoma, breast cancer or colon cancer.

In general, a LMW EPS derivative or a LMW sulfated EPS derivative, as described herein, or a pharmaceutical composition thereof, will be administered in an effective amount, *i.e.,* an amount that is sufficient to fulfill its intended purpose. The exact amount of the LMW EPS derivative or LMW sulfated EPS derivative, or pharmaceutical composition, to be administered will vary from subject to subject, depending on the age, sex, weight and general health condition of the subject to be treated, the desired biological or medical response and the like. In certain embodiments, an effective amount is one that delays or prevents the onset of cancer, and/or one that slows down or stops the progression, aggravation, or deterioration of the symptoms of cancer, and/or one that brings about amelioration of the symptoms of cancer, and/or one that prevents, delays and/or reduces the likelihood of occurrence of metastases formation and/or one that reduces the number, growth rate, size, etc... of metastases if metastases are already present in the subject. The effects of a treatment according to the invention may be monitored using any of the diagnostic assays, tests and procedures known in the art.

In certain embodiments, the LMW EPS derivative or LMW sulfated EPS derivative, or a pharmaceutical composition thereof, is administered alone in a method of treatment according to the present invention. In other embodiments, the LMW EPS derivative or LMW sulfated EPS derivative, or a pharmaceutical composition thereof, is administered in combination with at least one additional therapeutic agent or therapeutic procedure. The LMW EPS derivative or LMW sulfated EPS derivative, or pharmaceutical composition thereof, may be administered prior to administration of the therapeutic agent or therapeutic procedure, concurrently with the therapeutic agent or procedure, and/or following administration of the therapeutic agent or procedure.

Therapeutic agents that may be administered in combination with the LMW EPS derivative or LMW sulfated EPS derivative, or a pharmaceutical composition thereof, may be selected among a large variety of biologically active compounds that are known to have a beneficial effect in the treatment of cancer or to a patient in general (e.g., anti-cancer agents, anti-inflammatory agents, immunomodulatory agents, analgesics, antimicrobial agents, antibacterial agents, antibiotics, antioxidants, antiseptic agents, and combinations thereof). Therapeutic procedures that may be performed in combination with administration of the LMW EPS derivative or LMW sulfated EPS derivative, or pharmaceutical composition thereof, include, but are not limited to, surgery, radiotherapy, and the like.

Anti-cancer agents that may be administered in combination with the LMW EPS derivative or LMW sulfated EPS derivative, or a pharmaceutical composition thereof, include drugs conventionally classified in one of the following group: alkylating agents, purine antagonists, pyrimidine antagonists, plant alkaloids, intercalating antibiotics, aromatase inhibitors, anti-metabolites, mitotic inhibitors, growth factor inhibitors, cell cycle inhibitors, enzymes, topoisomerase inhibitors, biological response modifiers, anti-hormones and anti-androgens. Examples of such anti-cancer agents include, but are not limited to, BCNU, cisplatin, gemcitabine, hydroxyurea, paclitaxel, temozolomide, topotecan, fluorouracil, vincristine, vinblastine, procarbazine, decarbazine, altretamine, methotrexate, mercaptopurine, thioguanine, fludarabine phosphate, cladribine, pentostatin, cytarabine, azacitidine, etoposide, teniposide, irinotecan, docetaxel, doxorubicin, daunorubicin, dactinomycin, idarubicin, plicamycin, mitomycin, bleomycin, tamoxifen, flutamide, leuprolide, goserelin, aminogluthimide, anastrozole, amsacrine, asparaginase, mitoxantrone, mitotane and amifostine.

Other examples of such anti-cancer agents include therapeutic antibodies used in the treatment of cancer, including, but are not limited to, anti-CD52 antibodies such as alemtuzumab (CAMPATH^{™}), which is used in the treatment of chronic lymphocytic leukemia; anti-VEGF antibodies including bevacizumab (AVASTIN^{™}) which is used in the treatment of colorectal cancer and breast cancer; anti-CD33 antibodies, including gemtuzumab ozogamicin (MYLOTARG^{™}) which is used in the treatment of acute myeloid leukemia; anti-CD20 antibodies including ibritumomab (ZEVALIN^{™}) which is used in the treatment of lymphoma, rituximab (RITUXAN^{™}) which is used in the treatment of Hodgkin lymphoma, tositumomab (BEXXAR^{™}) which is used in the treatment of Hodgkin lymphoma and of atumumab (ARZERRA^{™}) which is used in the treatment of chronic lymphocytic leukemia; anti-EGFR antibodies such as cetuximab (ERBITUX^{™}) which is used in the treatment of colorectal cancer, head and neck cancer, and squamous cell carcinoma, and panitumumab (VECTIBEX^{™}) which is used in the treatment of colorectal cancer; anti-Her2 antibodies, including trastuzumab (HERCEPTIN^{™}) which is used in the treatment of breast cancer and stomach cancer; anti-CTLA4 antibodies including Ipilimumab (YERVOY^{™}) which is used in the treatment of melanoma; adnectins; and domain antibodies. Active fragments and fusions of these antibodies will also find use herein.

### B. Administration

A LMW EPS derivative or LMW sulfated EPS derivative (optionally after formulation with one or more appropriate pharmaceutically acceptable carriers or excipients), in a desired dosage can be administered to a subject in need thereof by any suitable route. Various delivery systems are known and can be used to administer a LMW EPS derivative or LMW sulfated EPS derivative of the present invention, including tablets, capsules, injectable solutions, encapsulation in liposomes, microparticles, microcapsules, etc. Methods of administration include, but are not limited to, dermal, intradermal, intramuscular, intraperitoneal, intralesional, intravenous, subcutaneous, intranasal, pulmonary, epidural, ocular, and oral routes. A LMW EPS derivative or LMW sulfated EPS derivative, or a pharmaceutical composition thereof, may be administered by any convenient or other appropriate route, for example, by infusion or bolus injection, by adsorption through epithelial or mucocutaneous linings (*e.g.,* oral, mucosa, rectal and intestinal mucosa, etc). Administration can be systemic or local. Parenteral administration may be directed to a given tissue of the patient, such as by catheterization. As will be appreciated by those of ordinary skill in the art, in embodiments where a LMW EPS derivative or LMW sulfated EPS derivative is administered along with an additional therapeutic agent, the LMW EPS derivative or LMW sulfated EPS derivative and the therapeutic agent may be administered by the same route (*e.g.,* orally) or by different routes (*e.g.,* orally and intravenously).

### C. Dosage

Administration of a LMW EPS derivative or LMW sulfated EPS derivative (or a pharmaceutical composition thereof) according to the present invention will be in a dosage such that the amount delivered is effective for the intended purpose. The route of administration, formulation and dosage administered will depend upon the therapeutic effect desired, the severity of the disorder being treated, the presence of any infection, the age, sex, weight and general health condition of the patient as well as upon the potency, bioavailability and *in vivo* half-life of the LMW EPS derivative or LMW sulfated EPS derivative, the use (or not) of concomitant therapies, and other clinical factors. These factors are readily determinable by the attending physician in the course of the therapy. Alternatively, or additionally, the dosage to be administered can be determined from studies using animal models. Adjusting the dose to achieve maximal efficacy based on these or other methods is well known in the art and is within the capabilities of trained physicians. As studies are conducted using a LMW EPS derivative or LMW sulfated EPS derivative, further information will emerge regarding the appropriate dosage levels and duration of treatment.

A treatment according to the present invention may consist of a single dose or multiple doses. Thus, administration of a LMW EPS derivative or LMW sulfated EPS derivative, or a pharmaceutical composition thereof, may be constant for a certain period of time or periodic and at specific intervals, e.g., hourly, daily, weekly (or at some other multiple day interval); monthly, yearly (*e.g.,* in a time release form). Alternatively, the delivery may occur at multiple times during a given time period, *e.g.,* two or more times per week, two or more times per month, and the like. The delivery may be continuous delivery for a period of time, e.g., intravenous delivery.

### III - Pharmaceutical Compositions

As mentioned above, the LMW EPS derivative or LMW sulfated EPS derivative described herein may be administered *per se* or as a pharmaceutical composition. Accordingly, the present invention provides pharmaceutical compositions comprising an effective amount of a LMW EPS derivative or LMW sulfated EPS derivative and at least one pharmaceutically acceptable carrier or excipient. In some embodiments, the composition further comprises one or more additional biologically active agents.

A LMW EPS derivative or LMW sulfated EPS derivative, or a pharmaceutical composition thereof, may be administered in any amount and using any route of administration effective for achieving the desired prophylactic therapeutic effect. The optimal pharmaceutical formulation can be varied depending upon the route of administration and desired dosage. Such formulations may influence the physical state, stability, rate of *in vivo* release, and rate of *in vivo* clearance of the administered active ingredient.

The pharmaceutical compositions of the present invention may be formulated in dosage unit form for ease of administration and uniformity of dosage. The expression "unit dosage form", as used herein, refers to a physically discrete unit suited as unitary dosages for the patient to be treated. It will be understood, however, that the total daily dosage of the compositions will be decided by the attending physician within the scope of sound medical judgement.

### A. Formulation

Injectable preparations, for example, sterile injectable aqueous or oleaginous suspensions may be formulated according to the known art using suitable dispersing or wetting agents, and suspending agents. The sterile injectable preparation may also be a sterile injectable solution, suspension or emulsion in a non-toxic parenterally acceptable diluent or solvent, for example, as a solution in 2,3-butanediol. Among the acceptable vehicles and solvents that may be employed are water, Ringer's solution, U.S.P. and isotonic sodium chloride solution. In addition, sterile, fixed oils are conventionally employed as a solution or suspending medium. For this purpose, any bland fixed oil can be employed including synthetic mono- or di-glycerides. Fatty acids such as oleic acid may also be used in the preparation of injectable formulations. Sterile liquid carriers are useful in sterile liquid form compositions for parenteral administration.

Injectable formulations can be sterilized, for example, by filtration through a bacterial-retaining filter, or by incorporating sterilizing agents in the form of sterile solid compositions which can be dissolved or dispersed in sterile water or other sterile injectable medium prior to use. Liquid pharmaceutical compositions which are sterile solutions or suspensions can be administered by, for example, intravenous, intramuscular, intraperitoneal or subcutaneous injection. Injection may be *via* single push or by gradual infusion. Where necessary or desired, the composition may include a local anesthetic to ease pain at the site of injection.

In order to prolong the effect of an active ingredient, it is often desirable to slow the absorption of the ingredient from subcutaneous or intramuscular injection. Delaying absorption of a parenterally administered active ingredient may be accomplished by dissolving or suspending the ingredient in an oil vehicle. Injectable depot forms are made by forming micro-encapsulated matrices of the active ingredient in biodegradable polymers such as polylactide-polyglycolide. Depending upon the ratio of active ingredient to polymer and the nature of the particular polymer employed, the rate of ingredient release can be controlled. Examples of other biodegradable polymers include poly(orthoesters) and poly(anhydrides). Depot injectable formulations can also be prepared by entrapping the active ingredient in liposomes or microemulsions which are compatible with body tissues.

Liquid dosage forms for oral administration include, but are not limited to, pharmaceutically acceptable emulsions, microemulsions, solutions, suspensions, syrups, elixirs, and pressurized compositions. In addition to the LMW EPS derivative or LMW sulfated EPS derivative, the liquid dosage form may contain inert diluents commonly used in the art such as, for example, water or other solvent, solubilising agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cotton seed, ground nut, corn, germ, olive, castor, and sesame oils), glycerol, tetrahydrofurfuryl alcohol, polyethylene glycols, and fatty acid esters of sorbitan and mixtures thereof. Besides inert diluents, the oral compositions can also include adjuvants such as wetting agents, suspending agents, preservatives, sweetening, flavouring, and perfuming agents, thickening agents, colors, viscosity regulators, stabilizes or osmo-regulators. Examples of suitable liquid carriers for oral administration include water (potentially containing additives as above, *e.g.,* cellulose derivatives, such as sodium carboxymethyl cellulose solution), alcohols (including monohydric alcohols and polyhydric alcohols such as glycols) and their derivatives, and oils (e.g., fractionated coconut oil and arachis oil). For pressurized compositions, the liquid carrier can be halogenated hydrocarbon or other pharmaceutically acceptable propellant.

Solid dosage forms for oral administration include, for example, capsules, tablets, pills, powders, and granules. In such solid dosage forms, the LMW EPS derivative or LMW sulfated EPS derivative described herein may be mixed with at least one inert, pharmaceutically acceptable excipient or carrier such as sodium citrate or dicalcium phosphate and one or more of: (a) fillers or extenders such as starches, lactose, sucrose, glucose, mannitol, and silicic acid; (b) binders such as, for example, carboxymethylcellulose, alginates, gelatine, polyvinylpyrrolidone, sucrose, and acacia; (c) humectants such as glycerol; (d) disintegrating agents such as agar-agar, calcium carbonate, potato or tapioca starch, alginic acid, certain silicates, and sodium carbonate; (e) solution retarding agents such as paraffin; absorption accelerators such as quaternary ammonium compounds; (g) wetting agents such as, for example, cetyl alcohol and glycerol monostearate; (h) absorbents such as kaolin and bentonite clay; and (i) lubricants such as talc, calcium stearate, magnesium stearate, solid polyethylene glycols, sodium lauryl sulfate, and mixtures thereof. Other excipients suitable for solid formulations include surface modifying agents such as non-ionic and anionic surface modifying agents. Representative examples of surface modifying agents include, but are not limited to, poloxamer 188, benzalkonium chloride, calcium stearate, cetostearyl alcohol, cetomacrogol emulsifying wax, sorbitan esters, colloidal silicon dioxide, phosphates, sodium dodecylsulfate, magnesium aluminum silicate, and triethanolamine. In the case of capsules, tablets and pills, the dosage form may also comprise buffering agents.

Solid compositions of a similar type may also be employed as fillers in soft and hard-filled gelatine capsules using such excipients as lactose or milk sugar as well as high-molecular-weight polyethylene glycols and the like. The solid dosage forms of tablets, dragees, capsules, pills, and granules can be prepared with coatings and shells such as enteric coatings, release controlling coatings and other coatings well known in the pharmaceutical formulating art. They may optionally contain opacifying agents and can also be of a composition such that they release the active ingredient(s) only, or preferably, in a certain part of the intestinal tract, optionally, in a delaying manner. Examples of embedding compositions which can be used include polymeric substances and waxes.

In certain embodiments, it may be desirable to administer an inventive composition locally to a specific area. This may be achieved, for example, and not by way of limitation, by local infusion during surgery, topical application, by injection, by means of a catheter, by means of suppository, or by means of a skin patch or stent or another implant.

For topical administration, the composition is preferably formulated as a gel, an ointment, a lotion, or a cream which can include carriers such as water, glycerol, alcohol, propylene glycol, fatty alcohols, triglycerides, fatty acid esters, or mineral oil. Other topical carriers include liquid petroleum, isopropyl palmitate, polyethylene glycol, ethanol (95%), polyoxyethylenemonolaurat (5%) in water, or sodium lauryl sulfate (5%) in water. Other materials such as antioxidants, humectants, viscosity stabilizers, and similar agents may be added as necessary.

In addition, in certain instances, it is expected that the inventive compositions may be disposed within transdermal devices placed upon, in, or under the skin. Such devices include patches, implants, and injections which release the active ingredient by either passive or active release mechanisms. Transdermal administrations include all administration across the surface of the body and the inner linings of bodily passage including epithelial and mucosal tissues. Such administrations may be carried out using the present compositions in lotions, creams, foams, patches, suspensions, solutions, and suppositories (rectal and vaginal).

Transdermal administration may be accomplished through the use of a transdermal patch containing an active ingredient (*i.e.,* a LMW EPS derivative or LMW sulfated EPS derivative described herein) and a carrier that is non-toxic to the skin, and allows the delivery of the ingredient for systemic absorption into the bloodstream *via* the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may be suitable. A variety of occlusive devices may be used to release the active ingredient into the bloodstream such as a semi-permeable membrane covering a reservoir containing the active ingredient with or without a carrier, or a matrix containing the active ingredient.

Suppository formulations may be made from traditional materials, including cocoa butter, with or without the addition of waxes to alter the suppository's melting point, and glycerine. Water soluble suppository bases, such as polyethylene glycols of various molecular weights, may also be used.

Materials and methods for producing various formulations are known in the art and may be adapted for practicing the subject invention. Suitable formulations for the delivery of antibodies can be found, for example, in *"*Remington's Pharmaceutical Sciences", E.W. Martin, 18th Ed., 1990, Mack Publishing Co.: Easton, PA.

### B. Additional Biologically Active Agents

In certain embodiments, the LMW EPS derivative or LMW sulfated EPS derivative is the only active ingredient in a pharmaceutical composition of the present invention. In other embodiments, the pharmaceutical composition further comprises one or more additional biologically active agents. Examples of suitable biologically active agents include, but are not limited to, anti-cancer agents, anti-inflammatory agents, immunomodulatory agents, analgesics, antimicrobial agents, antibacterial agents, antibiotics, antioxidants, antiseptic agents, and combinations thereof. Examples of specific anti-cancer agents, including anti-cancer antibody agents, have been listed above.

In such pharmaceutical compositions, the LMW EPS derivative or LMW sulfated EPS derivative and the at least one additional biologically active agent may be combined in one or more preparations for simultaneous, separate or sequential administration of the LMW EPS derivative or LMW sulfated EPS derivative and the biologically active agent(s). More specifically, an inventive composition may be formulated in such a way that the LMW EPS derivative or LMW sulfated EPS derivative and the biologically active agent(s) can be administered together or independently from each other. For example, a LMW EPS derivative or LMW sulfated EPS derivative and a biologically active agent can be formulated together in a single composition. Alternatively, they may be maintained (e.g., in different compositions and/or containers) and administered separately.

### C. Pharmaceutical Packs of Kits

In another aspect, the present invention provides a pharmaceutical pack or kit comprising one or more containers (e.g., vials, ampoules, test tubes, flasks or bottles) containing one or more ingredients of an inventive pharmaceutical composition, allowing administration of the LMW EPS derivative or LMW sulfated EPS derivative of the present invention.

Different ingredients of a pharmaceutical pack or kit may be supplied in a solid (*e.g.,* lyophilized) or liquid form. Each ingredient will generally be suitable as aliquoted in its respective container or provided in a concentrated form. Packs or kits according to the invention may include media for the reconstitution of lyophilized ingredients. Individual containers of the kits will preferably be maintained in close confinement for commercial sale.

In certain embodiments, a pack or kit includes one or more additional therapeutic agent(s). Optionally, associated with the container(s) can be a notice or package insert in the form prescribed by a governmental agency regulating the manufacture, use or sale of pharmaceutical or biological products, which notice reflects approval by the agency of manufacture, use or sale for human administration. The notice of package insert may contain instructions for use of a pharmaceutical composition according to methods of treatment disclosed herein.

An identifier, *e.g.,* a bar code, radio frequency, ID tags, etc., may be present in or on the kit. The identifier can be used, for example, to uniquely identify the kit for purposes of quality control, inventory control, tracking movement between workstations, etc.

Further aspects and advantages of this invention will be disclosed in the following figures and examples, which should be regarded as illustrative and not limiting the scope of this application.

### Examples

The following examples describe some of the preferred modes of making and practicing the present invention. However, it should be understood that the examples are for illustrative purposes only and are not meant to limit the scope of the invention. Furthermore, unless the description in an Example is presented in the past tense, the text, like the rest of the specification, is not intended to suggest that experiments were actually performed or that data were actually obtained.

The aim of the study was to analyze the effects of three different low-molecular-weight HE800 molecules: HE800DR, HE800DRS (or HE800DRS₂₀) and highly sulfated HE800DRS (or HE800DRS₃₀) at different concentrations on the viability and proliferation of cells of different tumor cell lines: MNNG/HOS (Osteosarcoma), A549 (Lung cancer), MDA-MB-231 (Breast cancer), A375 (melanoma) and Caco2 (colon cancer).

### Materials and Methods

### Chemicals

HE800DR (Mw = 20,000 Da, Sulfate (S)% = 0%), HE800DRS (Mw = 20,000 Da, S% = 6 or 7%, OSO₃⁻ = 20%) and highly sulfated HE800DRS (Mw = 20,000 Da, S% = 10%, OSO₃⁻ = 30%) were supplied in powder form by the LEMMMB laboratory of IFREMER and reconstituted in PBS to a final concentration of 5 mg/ml (Table 1), aliquoted and stored to -20°C.

**Table 1. HE800 derivatives used in the study.**

| **Reference** | **Name** | **Molecular weight (g/mol)** | **Sulfation (%)** |
|---|---|---|---|
| MAP5 | HE800DR | 20,000 | 0 |
| MAP6 | HE800DRS₂₀ | 20,000 | 20 |
| MAP7 | HE800DRS₃₀ | 20,000 | 3 |

### Tumor Cell Lines and Culturing Media

All human tumor cell lines used in the present study were obtained from the American Tissue Cell Collection (ATCC, Molsheim, France). Cells from the MNNG/HOS Osteosarcoma cell line, A375 melanoma cell line and Caco2 Colon cancer cell line were cultured with DMEM 4.5 g/L high glucose, pyruvate, non-glutamine from Gibco (Thermo-Fisher), supplemented with glutamine (Thermo-Fisher) and 5% of fetal bovine serum (FBS, Thermo-Fisher). Cells from the A549 cell line were cultured with DEMEM/F12 (Sigma Aldrich), supplemented with glutamine (Thermo-Fisher) and 5% of fetal bovine serum (FBS, Thermo-Fisher). Cells from the MDA-MB-231 cell line were cultured with L-15 media from Gibco (Thermo-Fisher), supplemented with glutamine (Thermo-Fisher) and 5% of fetal bovine serum (FBS, Thermo-Fisher). All experiments were carried out at 37°C in a humidity-saturated controlled atmosphere and 5% CO₂.

### MTT Cell Viability Test

Cell viability essay was performed by seeding, in triplicate, 3,000 cells of the indicated cell type par well (25 µL) with 25 µL of culture medium for 4 hours in a 96-well Tissue Culture Plate Flat bottom (Falcon) before adding 50 µL of a HE800 EPS derivative at concentrations of 1, 5, 10, 50, 100, 500 µg and 1 mg mL⁻¹.

The plate was incubated at 37°C in a humidity-saturated controlled atmosphere and 5% CO₂ for 72 hours. At three days of treatment, a volume of 10 µL of 5 mg/mL MTT (Sigma-Aldrich) was added and incubated for at least 3 hours at 37°C and 5% CO₂. After this time, the liquid is removed and 200 µL of DMSO were added to each well to dissolve the formed formazan crystals before proceeding to the colorimetric quantification with a multi-well spectrophotometer (Victor 3x from PerkinElmer) at the wavelength of 500-600 nm.

### Proliferation Assay

Cell proliferation was analyzed by xCELLigence technology (Agilent). Background was measured by adding 50 µL of corresponding media into an E-Plate view 96 (Chem Agilent). Before the beginning of cell treatment, cells were seeded in triplicate at 5,000 cells per well (50 µL) for 4 hours. Then, 100 µL of the HE800 EPS derivative was added at the concentrations of 100 and 500 µg mL⁻¹ for MNNG/HOS, A549 and MDA-MB-231 cells, and 25 ad 50 µg mL⁻¹ for A375 and Caco2 cells. The choice of these concentrations for each particular cell line was determined as a function of the IC₅₀ established by the MTT assay. Proliferation curves were normalized with respect to the time point of drug incorporation. The plate was monitored for 100 hours (for MNNG/HOS, A549 and MDA-MB-231 cells) or 120 hours (for A375 and Caco2 cells) using a RTCA instruments (Agilent and ACEA).

### Results

### Effect of HE800 EPS Derivatives in Human MNNG/HOS Osteosarcoma Cell Line Viability and Cell Proliferation

An MTT essay was used to determine the effect of the three exopolysaccharides in human MNNG/HOS osteosarcoma cell viability. A range of concentrations from 1 µg to 1 mg mL⁻¹ was tested for each molecule. HE800DR showed a classical dose/response profile with an inhibition of MNNG/HOS cell viability that went from a 70% at 1 mg mL⁻¹, with respect to the control, to no effect at the lowest concentration tested (1 µg mL⁻¹) (Figure 1A). A 50% cell viability inhibition was observed between 500 and 100 µg mL⁻¹. In the case of the HE800DRS exopolysaccharide, a similar dose/response effect was observed (Figure 1B) with a plateau of cell viability of 30% at dose concentrations of 1 mg mL⁻¹, 500 and 100 µg mL⁻¹. Then, cell viability increased progressively until 90% at a concentration of 1 µg mL⁻¹. An effect of 50% of cell viability inhibition was determined at doses between 50 and 10 µg mL⁻¹. In order to get a better resolution of the critical concentrations of HE800DRS, a more precise range of concentrations was tested (Figure 1C). As observed before, HE800DRS presented a dose/response profile with an IC₅₀ for cell viability closed to 25 µg mL⁻¹. The highly sulfated HE800DRS exopolysaccharide resulted in a less effective inhibition molecule of cell viability in MNNG/HOS cell line (Figure 1D). The MTT essay tended to a valley profile where only the concentration of 500 µg mL⁻¹ resulted in a maximum inhibition of cell viability of close to 40% with respect to the control. Higher or lower concentrations of the highly sulfated HE800DRS resulted in a cell viability of 80%, whereas lower concentrations (from 50 to 1 µg mL⁻¹) did not present any effect on cell viability.

MNNG/HOS cells proliferation in the presence of each of the HE800 EPS derivatives was analyzed by xCELLigence technology. For the three molecules, concentrations of 500 and 100 µg mL⁻¹ were used (Figures 1E-G). Those selected concentrations were based on results from MTT assays. For each curve, data was normalized (Normalized cell index) with respect to the time of exopolysaccharides treatment.

In agreement with the MTT assay, the HE800DR molecule presented an inhibition of MNNG/HOS cell proliferation (Figure IE). This inhibition was almost identical for the two concentrations tested. In the case of HE800DRS, while treatment at 500 µg mL⁻¹ resulted in a similar inhibition than HE800DR, treatment at a concentration of 100 µg mL⁻¹ resulted in a strong inhibition of cell proliferation compared to the other molecules and concentrations (Figure IF). This data was in agreement with the effect observed in MTT tests. For the highly sulfated HE800DRS, results of cell proliferation were also similar to the MTT test, showing a similar inhibition of cell proliferation than the HE800DR treatment did (Figure 1G).

### Effect of HE800 EPS Derivatives in Human A549 Cell Line Viability and Cell Proliferation

Similar to the experiments carried out with the osteosarcoma cell line described above, an MTT essay was used to determine the effect of the three exopolysaccharide derivatives in cell viability at the same concentration range. HE800DR presented a stronger impact in A549 cell viability compared to the osteosarcoma cell line (Figure 2A). A lower range of HE800DR concentrations was tested (Figure 2B). This test showed that cells from the A549 cell line were sensitive to lower concentrations of the molecule with a 50% of viability at 2.5 µg mL⁻¹. The HE800DRS exopolysaccharide showed a classical dose/response profile (Figure 2C) with an inhibition of 50% of the cell viability at a dose of 50 µg mL⁻¹. The highly sulfated HE800DRS exopolysaccharide also showed a dose/response profile that reached a plateau of non-effect at a HE800 EPS derivative concentration of 50 µg mL⁻¹ (Figure 2D).

In terms of A549 cells proliferation (analyzed by xCELLigence technology at concentrations of 500 and 100 µg mL⁻¹), the three molecules induced a small reduction of A549 cell proliferation at a dose of 100 µg mL⁻¹ (Figures 2E-G). However, the highly sulfated HE800DRS molecule produced a better slowdown of lung tumor cell proliferation at 500 µg mL⁻¹ (Figures 2G).

### Effect of HE800 EPS Derivatives in Human MDA-MB-23 Breast Cancer Cell Line Viability and Cell Proliferation

Here also, an MTT essay was used to determine the effect of the exopolysaccharide derivatives in cell viability of the human MDA-MB-231 breast cancer cell line. The three molecules exhibited similar behavior to those observed for the MNNG/HOS osteosarcoma cell line. HE800DR followed a dose/response profile with an inhibition of MDA-MB-231 cell viability that went from a 60% at 1 mg mL⁻¹, with respect to the control, to almost no effect at the lower concentration tested (1 µg mL⁻¹) (Figure 3A). A 50% of cell viability inhibition was observed between 500 and 100 µg mL⁻¹ (Figure 3A). HE800DRS induced an inhibition of more than 50% of cell viability at doses 500 µg and 1 mg mL⁻¹. Nevertheless, its negative impact on cell viability was reduced at concentrations up to 100 µg mL⁻¹ (Figure 3B). In the case of the highly sulfated HE800DRS exopolysaccharide, only MDA-MB-231 cell viability was compromised at the dose treatment of 500 µg mL⁻¹. Other tested concentrations resulted in no inhibition of the viability (Figure 3C).

xCELLigence assay showed that MDA-MB-231 cells proliferation was almost unaffected by treatment with the HE800 EPS derivatives (Figures 3D-F). Only the highly sulfated HE800DRS molecule at 500 µg mL⁻¹ produced a significant reduction in MDA-MB-231 cell proliferation (Figures 3F).

### Effect of HE800 EPS Derivatives in Human A375 Melanoma Cell Line Viability and Cell Proliferation

The analysis of the viability of cells from the A375 cell line in the presence of each exopolysaccharide derivative was performed using an MTT essay. With the exception of the concentration of 1 µg mL⁻¹, the treatment of human melanoma cells with HE800DR led to a strong reduction of cell viability (70%) for all concentrations tested (Figure 4A). In the case of cell treatment with the HE800DRS exopolysaccharide, the A375 cell viability was found to follow a dose/response profile with a maximum of cell viability inhibition at concentrations of 500 µg and 1 mg mL⁻¹ (Figure 4B). Doses lower than 50 µg mL⁻¹ of HE800DRS had not impact on A375 cells viability. As observed in osteosarcoma and breast tumor cell lines, the highly sulfated HE800DRS exopolysaccharide compromised cell viability at a dose treatment of 500 µg mL⁻¹. Lower concentrations resulted in no inhibition of the cell viability (Figure 4C).

The analysis of the proliferation of cells of the A375 cell line during exopolysaccharide treatment was perform using an xCELLigence assay with an EPS derivative concentration of 50 and 25 µg mL⁻¹ (Figure 4D-F). While the three compounds at the two different doses were able to reduce cell proliferation, the HE800DR exopolysaccharide produced a slightly better inhibition of the proliferation of the A375 cell line (Figure 4D).

### Effect of HE800 EPS Derivatives in Human Caco2 Colon Cell Line Viability and Cell Proliferation

The human Caco2 colon cell line was used as a model to study the effect of the three exopolysaccharides HE800DR, HE800 DRS and highly sulfated HE800DRS on tumor cell viability and proliferation. The results of MTT essays showed that in the case of the Caco2 cell line, the three molecules exhibited dose/response profile curves (Figure 5A-C). 50% of cell viability was obtained at a dose concentration of 50 µg mL⁻¹ for HE800DR and HE800DRS (Figure 5A-B). As the HE800DRS profile did not changed too much as a function of concentrations tested, a better concentration resolution was performed (Figure 5C). Data showed no significant variation of cell viability between all concentrations of HE800DRS tested. On the other hand, ten times more of the highly sulfated HE800DRS (500 µg mL⁻¹) was needed to reach a 50% inhibition of cell viability (Figure 5D).

Proliferation of cells of the Caco2 cell line was analyzed using the xCELLigence technology in the presence of each of the HE800 EPS derivative at concentrations of 50 and 25 µg mL⁻¹ (Figure 5E-G). A treatment with the highly sulfated HE800DRS was found to result in a better inhibition of the proliferation of Caco2 cells at both doses tested (Figure 5G). No significant differences were observed between the control and each of the two EPS derivatives HE800DR and HE800DRS, at any of the tested concentrations (Figures 5E and 5F).

### Conclusions

HE800DR reduced cell viability in all cancer cell lines tested. In particularly, the A549 lung cancer cell line was found to be highly sensitive to low concentrations of HE800DR. The viability of cells from the Melanoma A375 cell line was also compromised by the HE800DR exopolysaccharide.

HE800DR and HE800DRS were found to induce an important inhibitor effect on cell viability and cell proliferation in MNNG/HOS osteosarcoma cell line.

The highly sulfated HE800DRS was found to be the least efficient molecule to inhibit cell viability in all of the cancer cell lines tested. However, proliferation of cells from the MDA-MB-231 breast cancer and Caco2 colon cancer cell lines showed a reduction of cell proliferation at high doses of the exopolysaccharide.

Throughout this application, various references describe the state of the art to which this invention pertains. The disclosures of these references are hereby incorporated by reference into the present disclosure.

## Claims

1. A low-molecular-weight exopolysaccharide derivative for use in the treatment of cancer in a subject, wherein said low-molecular-weight exopolysaccharide derivative is obtained by a method comprising:
(a) a step consisting of free-radical depolymerization of a marine native exopolysaccharide (EPS) from the strain HE800 of the *Vibrio diabolicus* genus so as to obtain a depolymerized EPS having a molecular weight of 5,000 to 100,000 g/mol; and
(b) a subsequent step consisting of isolating a low-molecular-weight exopolysaccharide derivative from the depolymerized EPS, wherein the low-molecular-weight exopolysaccharide derivative has a molecular weight comprised between 5,000 and 30,000 g/mol, preferably between 10,000 and 25,000 g/mol, more preferably between 15,000 and 22,000 g/mol, and even more preferably a molecular weight of about 20,000 g/mol;
or wherein said low-molecular-weight exopolysaccharide derivative is a low-molecular-weight sulfated exopolysaccharide derivative and said low-molecular-weight sulfated exopolysaccharide derivative is obtained by a method comprising:
(a') a step consisting of free-radical depolymerization of a marine native exopolysaccharide (EPS) from the strain HE800 of the *Vibrio diabolicus* genus so as to obtain a depolymerized EPS having a molecular weight of 5,000 to 100,000 g/mol;
(b') a subsequent step consisting of sulfation of the depolymerized EPS to obtain an sulfated depolymerized EPS, comprising adding to the depolymerized EPS at least one sulfation agent in an amount sufficient to obtain a sulfated polysaccharide having a degree of sulfate-group substitution of between 5% and 40% by weight relative to the total weight of the sulfated depolymerized EPS; and
(c') a subsequent step consisting of isolating the low-molecular-weight sulfated exopolysaccharide derivative from the sulfated depolymerized EPS, wherein the low-molecular-weight sulfated exopolysaccharide derivative has a molecular weight comprised between 5,000 and 30,000 g/mol, preferably between 10,000 and 25,000 g/mol, more preferably between 15,000 and 22,000 g/mol, and even more preferably a molecular weight of about 20,000 g/mol.

2. The low-molecular-weight exopolysaccharide derivative for the use according to claim 1, wherein step (b) or step (c') is carried out by fractionation, in particular fractionation performed by size exclusion chromatography.

3. The low-molecular-weight exopolysaccharide derivative for the use according to claim 1 or claim 2, wherein the low-molecular-weight exopolysaccharide derivative is HE800DR, which has a molecular weight of 20,000 g/mol and a degree of sulfate-group substitution of 0% by weight relative to the total weight of the sulfated depolymerized EPS.

4. The low-molecular-weight exopolysaccharide derivative for the use according to claim 1 or claim 2, wherein the low-molecular-weight exopolysaccharide derivative is the low-molecular-weight sulfated exopolysaccharide derivative, HE800DRS₂₀, which has a molecular weight of 20,000 g/mol and a degree of sulfate-group substitution of 20% by weight relative to the total weight of the sulfated depolymerized EPS.

5. The low-molecular-weight exopolysaccharide derivative for the use according to claim 1 or claim 2, the low-molecular-weight exopolysaccharide derivative is the low-molecular-weight sulfated exopolysaccharide derivative, HE800DRS₃₀, which has a molecular weight of 20,000 g/mol and a degree of sulfate-group substitution of 30% by weight relative to the total weight of the sulfated depolymerized EPS.

6. The low-molecular-weight exopolysaccharide derivative for the use according to any one of claims 1 to 5, wherein the cancer is selected from the group consisting of carcinoma, lymphoma, blastoma, sarcoma, and leukemia.

7. The low-molecular-weight exopolysaccharide derivative for the use according to any one of claims 1 to 6, wherein the cancer is selected from the group consisting of bone cancer, lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, and pituitary adenoma.

8. The low-molecular-weight exopolysaccharide derivative for the use according to any one of claims 1 to 6, wherein the cancer is selected from the group consisting of osteosarcoma, lung cancer, breast cancer, melanoma and colon cancer.

9. The low-molecular-weight exopolysaccharide derivative for the use according to any one of claims 1 to 8, wherein the subject is a cancer patient.

10. A pharmaceutical composition comprising an effective amount of a low-molecular-weight exopolysaccharide derivative as defined in any one of claims 1 to 5 and at least one pharmaceutically acceptable carrier or excipient for use in the treatment of cancer in a patient.

11. The pharmaceutical composition for the use according to claim 10, wherein the cancer is selected from the group consisting of carcinoma, lymphoma, blastoma, sarcoma, and leukemia.

12. The pharmaceutical composition for the use according to claim 10 or claim 11, wherein the cancer is selected from the group consisting of bone cancer, lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, and pituitary adenoma.

13. The pharmaceutical composition for the use according to claim 10 or claim 11, wherein the cancer is selected from the group consisting of osteosarcoma, lung cancer, breast cancer, melanoma and colon cancer.

14. The pharmaceutical composition for the use according to any one of claims 10 to 13, wherein the subject is a cancer patient.
